Europäisches Patentamt

(19) European Patent Office

Office européen des brevets

(11) Publication number : **0 341 004 B1**

(12) # EUROPEAN PATENT SPECIFICATION

(45) Date of publication of patent specification :
25.11.92 Bulletin 92/48

(21) Application number : **89304363.8**

(22) Date of filing : **28.04.89**

(51) Int. Cl.⁵ : **C07C 43/12, C07C 41/22**

(54) **A method of preparing 2-(difluoromethoxy)-1,1,1,2-tetrafluoroethane.**

(30) Priority : **06.05.88 US 190971**

(43) Date of publication of application :
**08.11.89 Bulletin 89/45**

(45) Publication of the grant of the patent :
**25.11.92 Bulletin 92/48**

(84) Designated Contracting States :
**DE FR GB IT**

(56) References cited :
**EP-A- 0 285 237**
**US-A- 3 897 502**

(73) Proprietor : **Anaquest, Inc.**
**110 Allen Road**
**Liberty Corner, NJ 07938-0804 (US)**

(72) Inventor : **Robin, Mark L.**
**245 East Golf Avenue**
**South Plainfield New Jersey 07080 (US)**
Inventor : **Halpern, Donald F.**
**156 Marian Avenue**
**Fanwood New Jersey 07023 (US)**

(74) Representative : **Dipl.-Phys.Dr. Manitz**
**Dipl.-Ing., Dipl.-W.-Ing. Finsterwald Dipl.-Ing.**
**Grämkow Dipl.-Chem.Dr. Heyn Dipl.-Phys.**
**Rotermund**
**Morgan, B.Sc.(Phys.) Robert-Koch-Strasse 1**
**W-8000 München 22 (DE)**

Jouve, 18, rue Saint-Denis, 75001 PARIS

## Description

The present invention relates to a method of preparing 2-(difluoromethoxy)-1,1,1,2-tetrafluoroethane.

EP-A-285 237 discloses the use of 2-(difluoromethoxy)-1,1,1,2-tetrafluoroethane as an anaesthetic.

2-(difluoromethoxy)-1,1,1,2-tetrafluoroethane or $CHF_2OCHFCF_3$, is also disclosed in Example XXI of US-A-3 897 502.

The compound 2-(difluoromethoxy)-1,1,1,2-tetrafluoroethane is normally a clear, colorless liquid with a slight non-pungent odor. It has the following physical properties: boiling point 23.5°C, molecular weight 168, vapor pressure (est) 660 mm Hg at 20°C, and specific gravity 1.44. The IR shows a prominent peak at 4903 and the $^1$H NMR shows a triplet at 6.5 ppm (J=70Hz) and a doublet of quartets at 5.9 ppm ($J_{gem}$=56Hz, $J_{vic}$=3Hz). The compound is non-flammable, and soda lime stable.

EP-A-285 237 discloses preparing this compound in 62% yield by reacting isoflurane ($CHF_2OCHClCF_3$) with bromine trifluoride using a procedure in which 2 ml of bromine trifluoride is added over 2 1/2 hrs to 15.5 g of isoflurane and the reaction maintained at about 14 to 18°C.

The starting material isoflurane (2-(difluoromethoxy)- 1,1,1-trifluoro-2-chloroethane) may be prepared by the methods disclosed in US-A-3 535 388, incorporated herein by reference. Isoflurane is also commercially available from Anaquest Division of BOC, Inc. (Madison, Wisconsin).

We have now found that if the reverse procedure (addition of isoflurane to bromine trifluoride) is adopted near quantitative yields of $CHF_2OCHFCF_3$ can be attained particularly at a molar ratio of $BrF_3$ to isoflurane of about 0.3:1. High percentage conversions can also be achieved.

According to the present invention there is provided a method of preparing 2-(difluoromethoxy)-1,1,1,2-tetrafluoroethane comprising reacting isoflurane with bromine trifluoride wherein the isoflurane is added to the bromine trifluoride.

The rapid rate of reaction of bromine trifluoride with isoflurane renders its control extremely easy. For example, when isoflurane addition is interrupted, the reaction is immediately halted and there is an immediate drop in reaction temperature. The reaction temperature is preferably maintained between 5 and 50°C. The ease of control, relatively simple equipment required and the high yields attainable make the process attractive as an industrial preparation. For reactions on the kilogram scale, an average 81% conversion and 95% yield are attainable.

Bromine trifluoride ($BrF_3$) is a hazardous material, exploding upon contact with water or organic materials. It can be safely handled by careful engineering design and adherence to proper operating procedures. At the present time bromine trifluoride is commercially available from Air Products and Chemicals, Inc. (Allentown, PA.)

To illustrate the present invention further, the following examples are provided, wherein the following definitions are employed:

$$\% \text{ conversion} = \frac{\text{moles product}}{\text{moles starting material fed}} \times 100$$

$$\% \text{ yield} = \frac{\text{moles product}}{\text{moles starting material consumed}} \times 100$$

GC = gas chromatograph

$t_r$ = retention time (minutes) from injection point

The structure of $CF_3CHFOCF_2H$ may be confirmed by the following instrumental data:

$^1$H NMR: doublet of quartets at = 5.9ppm,

$J_{OCHF}$ = 54.3Hz

$J_{CHF-CF_3}$ = 2.8Hz

triplet at = +6.5ppm, $J_{CF_2H}$ = 70.4Hz

$^{19}$F NMR: $\emptyset_{CF_2H}$ = -86.1ppm

$\emptyset_{CHF}$ = -146.5ppm

$\emptyset_{CHF_3}$ = -84.5ppm

$^4J_{F-F(CF_2HOCHF)}$ = 5.8Hz

$J_{F-F(CF_2)}$ = 160.3Hz

$J_{F-H(CF_2H)}$ = 70.0Hz

$J_{F-H(CHF)}$ = 55.3Hz

Mass Spectrum (electron impact):

Format: m/e (intensity), fragment id

149(1)$C_3H_2F_5O$, M-F; 101(17)$C_2HF_4$;

69(9)CF$_3$; 51(100)CF$_2$H; 32(9)CHF; 31(18)CF

## Example 1

A 200 ml Teflon reactor attached to a caustic scrubber was charged with 183.5 g (1.3 moles) of bromine trifluoride and the reactor lid attached. The bromine trifluoride charge was then stirred and 713.1 g (3.9 moles) of isoflurane added over a four hour period. The reactor temperature was maintained at 25-30°C by external cooling of the reactor with dry ice/carbon tetrachloride. The caustic scrubber (1.2 L of water, 304 g NaOH) was kept at -10 to -15°C throughout the run. Following the addition of isoflurane, the reactor was allowed to stand at room temperature for one hour. Excess isoflurane was then distilled into the scrubber by heating the reactor to 55°C. The contents of the scrubber were then poured into a cold separatory funnel and the organic layer drained off and washed with cold water.

Gas chromatographic analyses were performed on a GOW-MAC Model HP5790 having a thermal conductivity detector (TCD) and HP 3392A integrator. The column was 1% SP1000 on 60/80 Carbopack B, 1/8″ diameter X 20′. A flow rate of 60 cc/min, an injector temperature of 214°C, an oven temperature of 190°C and a detector temperature of 214°C was maintained. All GC results were reported in area %.

GC analysis of the isolated material (565.2 g) showed it to be 98.0% CHF$_2$OCHFCF$_3$ (t$_r$=3.2 minutes) and 1.6% isoflurane (t$_r$=6.13 minutes). The percent conversion was thus 85% (87% yield). Some losses of the highly volatile product (b.p. 23.5°C) occurred during the caustic work-up, which accounts for the less than quantitative results.

Attempts to moderate the reaction via the use of CCl$_4$ as a solvent produced no detectable amounts of CHF$_2$OCHFCF$_3$, but instead resulted in reaction with the solvent to produce chlorofluorocarbons (ie CFCl$_3$, CF$_2$Cl$_2$). The reaction of isoflurane with BrF$_3$ is likely ionic in nature, and the presence of elemental bromine was shown to have no effect on the reaction. Reaction of isoflurane with the less reactive interhalogen compound IF$_5$ produced only small amounts of product.

## Example 2

The following Table I summarizes the results of three experimental runs and show the superiority of the invention over the reverse procedure described in EP-A-285 237.

Table I: Reaction of Isoflurane with Bromine Trifluoride

| Run No. | Reactants (molar ratio) | Temp (C°) | Reaction Time (Hrs) | % Conversion | % Yield | Reaction Condition |
|---------|-------------------------|-----------|---------------------|--------------|---------|--------------------|
| 1 | Isoflurane: BrF$_3$ (3:1) | 25-30 | 6 | 89 | 99 | Addition of Isoflurane to BrF$_3$ |
| 2 | Isoflurane: BrF$_3$ (3:1) | 25-30 | 3 | 85 | 87 | Addition of Isoflurane to BrF$_3$ |
| 3 | Isoflurane: BrF$_3$ (3:1) | 25-30 | 5 | 79 | 79 | Presence of Br$_2$;Addition of Isoflurane to BrF$_3$ |

Claims

1. A method of preparing 2-(difluoromethoxy)-1,1,1,2-tetrafluoroethane comprising reacting isoflurane with bromine trifluoride, characterised in that the isoflurane is added to the bromine trifluoride.

2. A method according to claim 1, wherein isoflurane is added to bromine trifluoride in a ratio of 0.3 moles of bromine trifluoride to 1 mole of isoflurane.

3. A method according to claim 1 or claim 2, wherein said reaction is at a temperature to 5 to 50°C.

4. A method according to claim 3, wherein said reaction is at a temperature of about 30°C.

5. A method according to any one of the preceding claims, wherein the isoflurane is added to the bromine trifluoride over a period of from 3 to 6 hours.

Patentansprüche

1. Verfahren zur Herstellung von 2-(Difluormethoxy)-1,1,1,2-tetrafluorethan durch Umsetzung von Isofluran mit Bromtrifluorid, dadurch gekennzeichnet, daß das Isofluran zu dem Bromtrifluorid gegeben wird.

2. Verfahren nach Anspruch 1, dadurch gekennzeichnet, daß Isofluran zu Bromtrifluorid in einem Verhältnis von 0,3 Mol Bromtrifluorid zu 1 Mol Isofluran gegeben wird.

3. Verfahren nach Anspruch 1 oder Anspruch 2, dadurch gekennzeichnet, daß die Umsetzung bei einer Temperatur von 5 zu 50°C durchgeführt wird.

4. Verfahren nach Anspruch 3, dadurch gekennzeichnet, daß die Umsetzung bei einer Temperatur von etwa 30°C durchgeführt wird.

5. Verfahren nach einem der vorausgehenden Ansprüche, dadurch gekennzeichnet, daß Isofluran zum Bromtrifluorid während einer Zeit von 3 bis 6 Stunden zugegeben wird.

Revendications

1. Procédé pour la préparation de 2-(difluorométhoxy) -1,1,1,2-tétrafluoro-éthane, comprenant une étape consistant à faire réagir de l'isoflurane avec du trifluorure de brome, caractérisé en ce que l'isoflurane est ajouté au trifluorure de brome.

2. Procédé selon la revendication 1, caractérisé en ce que l'isoflurane est ajouté au trifluorure de brome dans un rapport de 0,3 moles de trifluorure de brome pour 1 mole d'isoflurane.

3. Procédé selon l'une ou l'autre des revendications 1 et 2, caractérisé en ce que ladite réaction est menée à une température de 5 à 50 °C.

4. Procédé selon la revendication 3, caractérisé en ce que ladite réaction est menée à une température d'environ 30 °C.

5. Procédé selon l'une quelconque des revendications précédentes, caractérisé en ce que l'isoflurane est ajouté au trifluorure de brome sur une période de 3 à 6 heures.